# EUROPEAN PATENT SPECIFICATION

(11) **EP 1 627 648 B1**
(45) Date of publication and mention of the grant of the patent: **11.08.2010**
(21) Application number: 03748150.4
(22) Date of filing: 18.09.2003
(51) Int. Cl.: A61L 9/12, B60H 3/00

(54) **VEHICLE AIR FRESHENER DIFFUSER**
FAHRZEUG-LUFTERFRISCHER-DIFFUSOR
DIFFUSEUR DE PARFUMS POUR VEHICULES

(30) Priority: 16.04.2003 WO PCT/ES03/00179
(43) Date of publication of application: 22.02.2006
(73) Proprietor: Zobele España, S.A., 08290 Cerdanyola del Vallés (ES)
(72) Inventor: CASERTA, Andrea, 08290 Cerdanyola Del Valles (ES); GARCIA FABREGA, Ruben, 08290 Cerdanyola Del Valles (ES); ALVAREZ LEAL, Xavier, 08290 Cerdanyola Del Valles (ES); BASAGANAS MILLAN, Jordi, 08290 Cerdanyola Del Valles (ES)
(74) Representative: Carpintero Lopez, Francisco
(86) International application number: PCT/ES2003/000467
(87) International publication number: WO 2004/091673

(56) References cited:
- EP-A1- 1 031 446
- EP-A1- 1 195 277
- ES-U- 283 811
- ES-U- 1 050 062
- US-A- 4 154 398

## Description

### OBJECT OF THE INVENTION

The present invention relates to a diffuser of air-freshening or aromatic products, specially conceived to be used inside a motor vehicle, fitted with means of fastening to a diffuser grill of the air conditioning system of the vehicle, and fitted at the same time with means to adjust at the will of the user the flow of air that passes through the diffuser and, as a consequence, the quantity of air-freshening product delivered to the atmosphere per unit of time.

The object of the invention is to achieve optimum functional benefits from the diffuser, in the aspects that have just been mentioned, whilst at the same time having maximum structural simplicity.

### BACKGROUND OF THE INVENTION

Diffusers are known from historical times which use a small recipient container of the air-freshening product in question, in the form of a bottle, from the mouth of which projects a wick through which ascends said product by capillary action, so that the air which is circulating and comes into contact with the projecting sector of said wick causes a rapid evaporation of the product and its consequent passage into the atmosphere. Also known from historical times is the controlling of the flow of air through the wick in some manner, to control at the same time the delivery level of the product into the atmosphere.

In said sense mention is made of the patent US 4.621.768, in which the wick is surrounded, in its sector projecting from the recipient or flask, through a cylindrical neck, fitted with wide side vents for passage of the air and with a screw thread for axial displacement of a cap, in turn coaxially facing the wick, so that when said cap undergoes a rotational movement, by acting on the casing of the diffuser, an axial displacement of the aforementioned cap takes place, which is capable of blocking in a greater or lesser extent, even blocking fully, the vents of the aforementioned neck, regulating thereby the circulation of air through the same.

It is also known, through the European patent EP 1 031 446, that when the diffuser is intended to be located inside an automobile, it is mounted on one of the grills of the vehicle air-conditioning equipment, so that the circulation of air over the wick is a function not only of the degree of opening of the container thereof, but also of the speed of the fan of the vehicle which impels the air toward the aforementioned diffuser grill.

In a more specific way in the European patent mentioned, the control of air flow is regulated by means of a cap which covers the wick and which is moveable axially with respect thereto with the collaboration of a rack and a small pinion, this last item being associated with an externally operated control.

For its part and for the fastening of the diffuser to the diffuser grill of the vehicle, it has been foreseen that the casing of the first incorporates a clip with four arms in that which is to be its rear face, so that said clip is able to grasp a slat of the diffuser grill of the vehicle, both in the case where this adopts a horizontal position and when it is situated vertically, the diffuser maintaining a correct working position, in any case.

### DESCRIPTION OF THE INVENTION

The diffuser which the invention discloses has been designed and structured in order to improve the aforementioned features, both from the point of view of regulation and emission capacity of air-freshening product per unit of time, and in that which relates to its fastening to the diffuser grill of the air-conditioning equipment of the vehicle.

For this and in more specific terms, the diffuser disclosed is constituted by means of two half-casings, a front one and another rear one, capable of coupling to each other by matched engagement and each one of them provided with a slatted and intermediate sector, for passage of the air, both casings defining as a whole, in assembled position, a cylindrical lower neck, in which is tightly coupled the neck of the recipient container of the air-freshening product, so that the wick of this last item is facing the slatted sectors or grills, and another neck above, coaxial with the lower one and with the actual wick of the recipient, this upper neck coming with a peripheral and internal rib which runs in a groove of a plug which projects upwardly from the casing configuring an upper manually operated knob, and provided at the bottom with two skirts, in diametrical opposition, capable of blocking in a greater or lesser extent the slatted sectors or grills of the half-casing, with the possibility that one of the skirts is cut, so that the length of this cut skirt is approximately three times less than that of the other skirt, with the special particularity that in the intermediate situation of opening the open slatted sectors are offset with respect to the intermediate wick, forcing the air to follow a longer path, specifically a winding path or encircling the wick, which improves the evaporation conditions.

The plug incorporates in the groove mentioned a widened sector, which affects approximately a quadrant thereof, in which runs a butt established in one of the two half-casings, to limit thereby the end positions of fully open and fully closed. This plug is operated manually from above and it can incorporate indications which facilitate viewing the degree of opening of the evaporator.

Likewise the form of the grills of the front half-casing, allows viewing and displacement of the plug element, which facilitates a rapid viewing of the degree of opening of the same without the necessity of seeing any other graphic printed on the plug.

In accordance with another of the characteristics of the invention the rear half-casing incorporates, as a means of fastening to the diffuser grill of the air-conditioning equipment of the vehicle, a clip provided exclusively with two arms, which signifies a remarkable simplification in the same, without prejudice to the features thereof, since it has been foreseen that said clip is mounted with removable character on the aforementioned half-casing, it being consequently possible to adopt a horizontal or vertical position, according to the necessities of each case. In addition this structural simplification, the absence in the clip of two inoperative arms, also eliminates the problem of double clips, wherein it is necessary to eliminate by breaking in two of said arms, to prevent them causing an obstruction by resting on the slat or partition of the diffuser grill.

### DESCRIPTION OF THE DRAWINGS

To complete the description being made and with the object of assisting in a better understanding of the characteristics of the invention, in accordance with a preferred example of practical embodiment thereof, accompanying as an integral part of said description, is a set of drawings wherein, by way of illustration and not restrictively, the following has been represented:

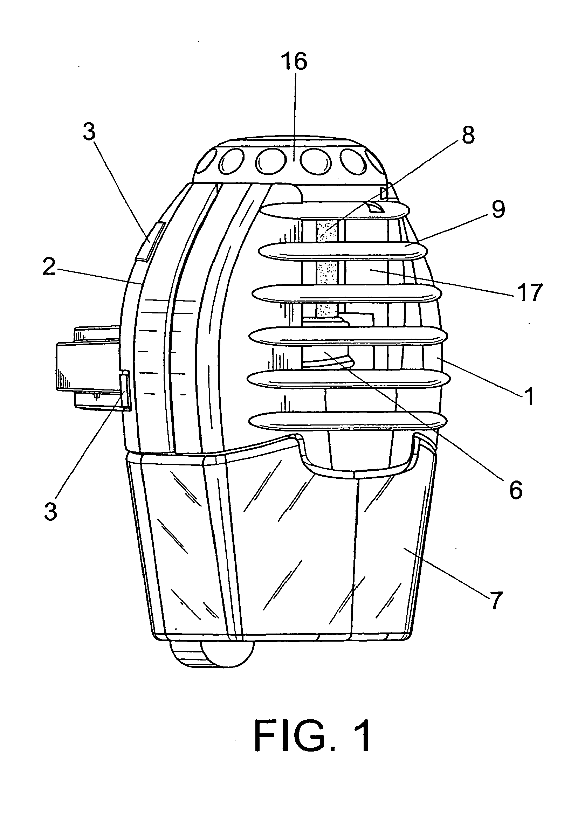

Figure 1 shows a frontal corner view in perspective of a diffuser of air-freshening products for vehicles implemented in accordance with the object of the present invention.

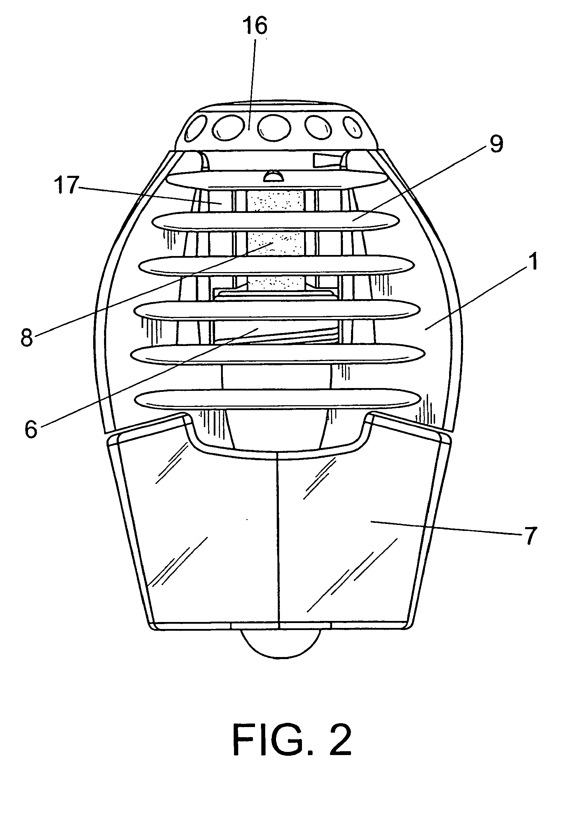

Figure 2 shows a view in frontal elevation of the same.

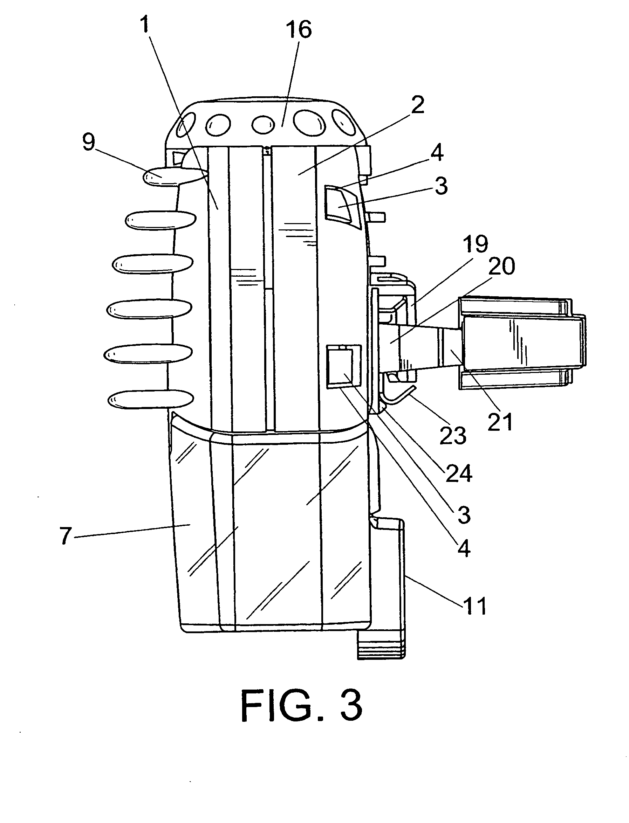

Figure 3 shows a profile.

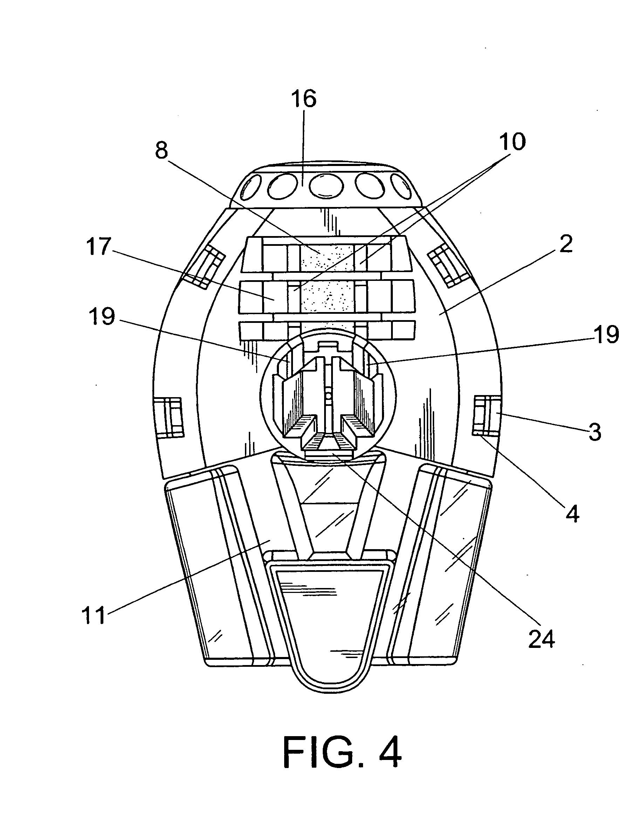

Figure 4 shows a view in rear elevation in which the two skirts of the plug have the same length.

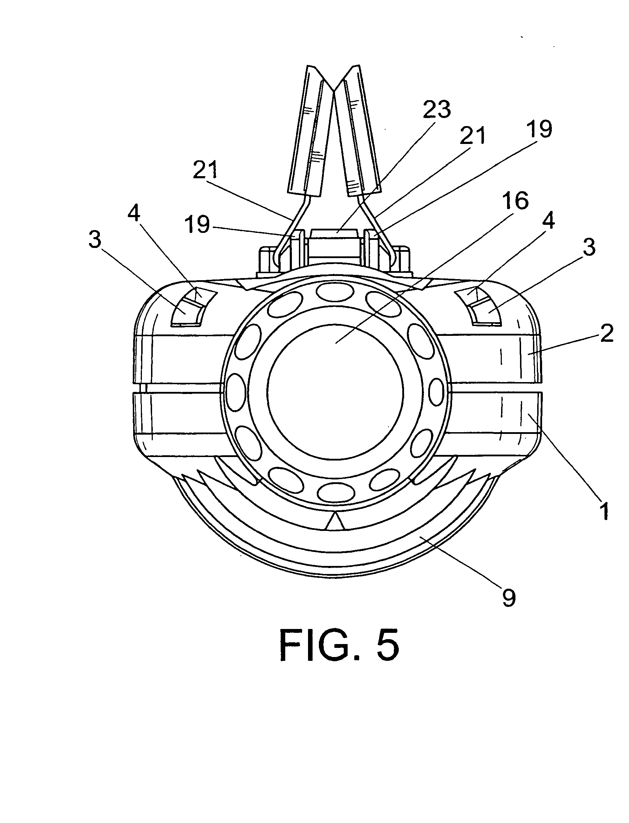

Figure 5 shows a top plan view.
Figures 6 and 7 show two views in frontal elevation of the diffuser, with its front half-casing removed, the first one in the fully open situation and the second in the situation of fully closed.
Figure 8 shows a detail in side elevation of the plug with the two skirts having the same length.
Figure 9 shows a detail in front elevation of the rear half-casing with the two skirts having a different length.

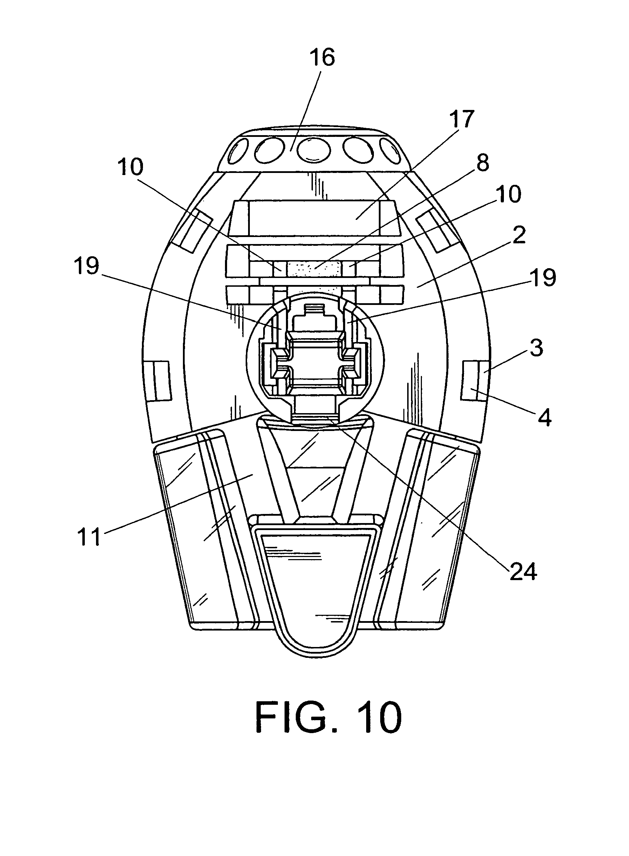

Figure 10 shows a detail in rear elevation of the two skirts with different lengths.

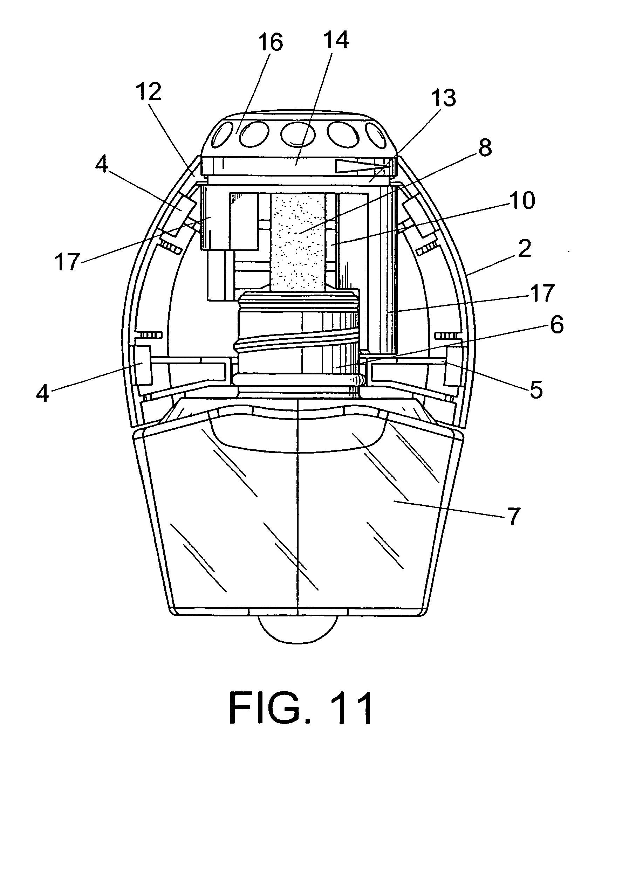

Figure 11 shows a front elevation view of the diffuser, without its front half-casing in a full aperture position in which the skirts have a different length.
Figure 12 shows a front elevation view of the rear half-casing.
Figure 13 shows a detail in perspective of the rear half-casing, now from its rear or external face and at the level of the means of fastening of the clip.

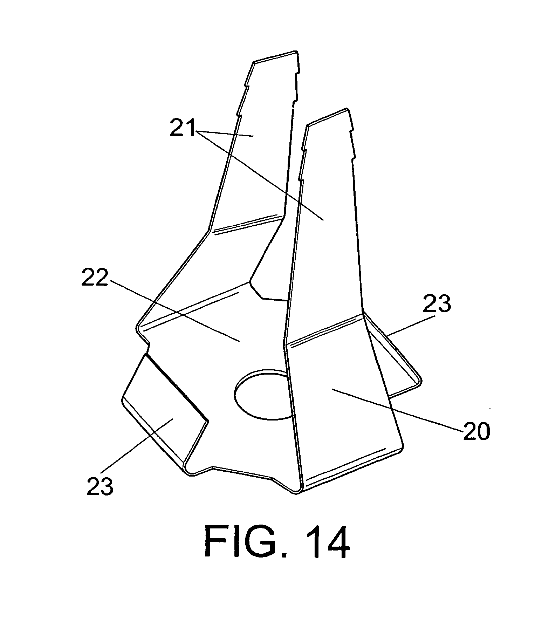

Figure 14 shows, finally, a detail in perspective of the mentioned clip.

### PREFERRED EMBODIMENT OF THE INVENTION

In the light of the figures mentioned, it can be observed how the diffuser that the invention discloses is constituted by means of two half-casings, one frontal (1) and another rear (2), obtained by means of injection moulding based on plastic material, the front casing (1) having four elastically deformable legs (3), through which it is coupled by matched engagement in respective vents (4) of the rear half-casing (2), so that in the engagement between the two half-casings (1) and (2), a lower cylindrical neck (5) is defined for close coupling of the neck (6) of the recipient container (7) of the air-freshening product, from which neck (6) projects the classic wick (8) which is housed axially inside the casing (1-2), and which absorbs the air-freshening product, so that it is carried by the air current which traverses the casing, between two grills (9) and (10), present respectively in the front half-casing (1) and in the rear half-casing (2), properly centred and facing each other, which recipient (7) is stabilized in the context of the diffuser with the collaboration of a retention arm (11), a lower continuation of the rear half-casing (2).

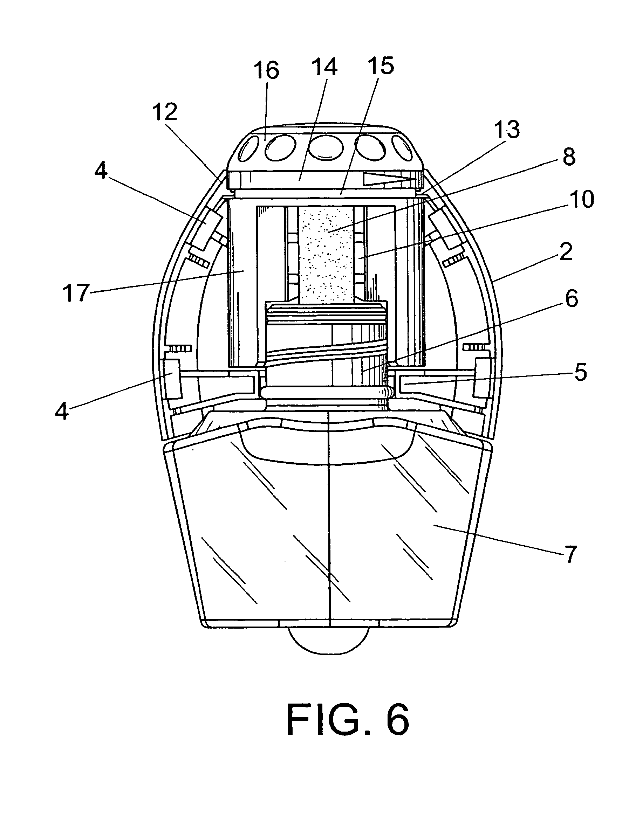

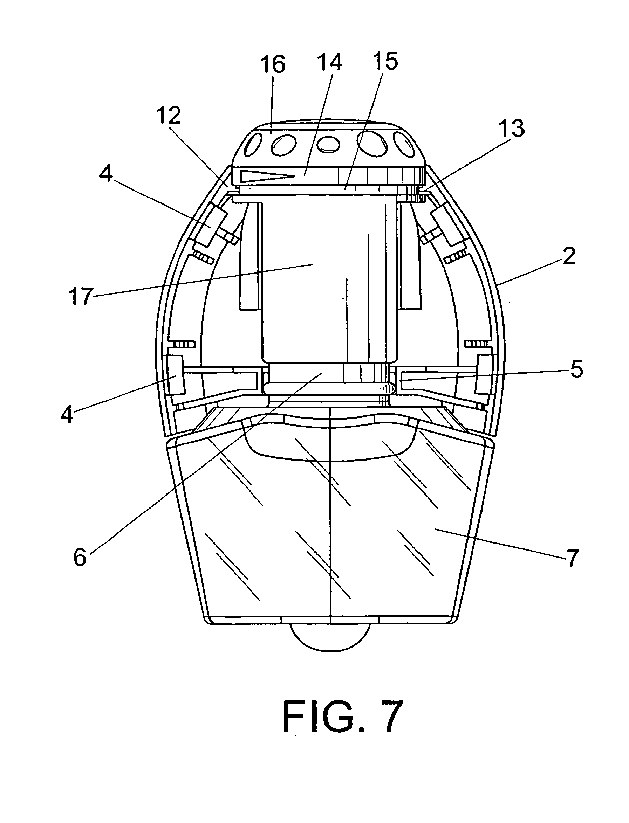

Additionally the half-casings (1) and (2) configure a second neck (12), at the level of the upper end, in which a peripheral rib (13) is established for coupling, with the possibility of rotation, of a plug (14), provided with a groove (15) for coupling with the rib (13), which plug is finished outwardly in a projecting knob (16), for manual operation of the same, and provided at the bottom or internally with a pair of skirts (17), included in an imaginary cylindrical plane, capable of being offset with respect to the grills (9) and (10), as shown by the position of figure 6, of closing completely said grills, just as occurs in the position shown in figure 7, or of adopting any intermediate position.

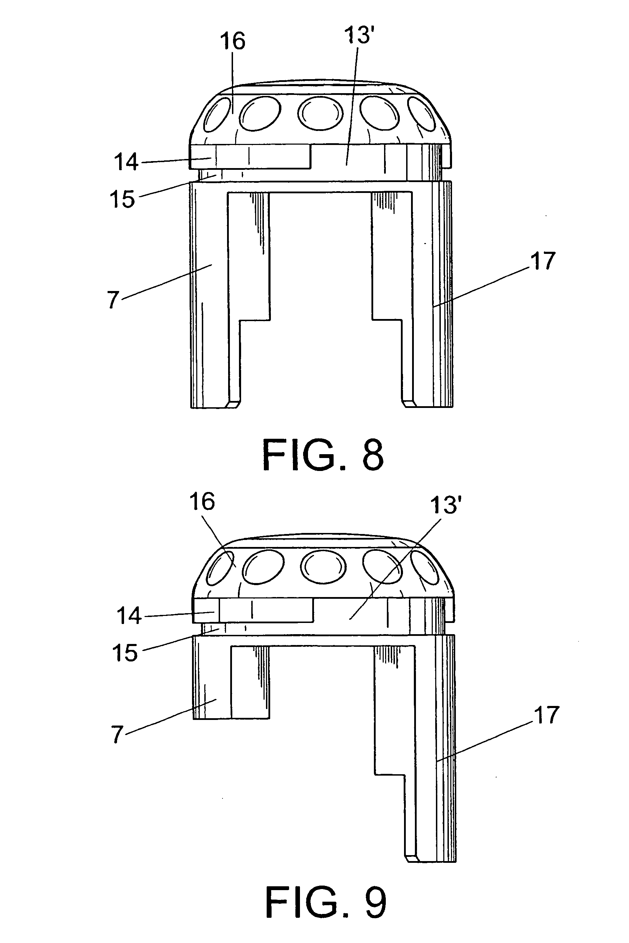

It is possible for one of the skirts (17) to be cut, so that the length of this cut skirt (17) is approximately three times less than the length of the other skirt (17), as shown in figure 9, being capable of assuming any position with respect to the grills (9) and (10).

The opening and closing limiting positions of the diffuser, more specifically the limiting positions of the plug (14), are defined by a widened stretch (13') of the groove (13), in which runs a butt (18) projecting inwardly from the neck (12) of the rear half-casing (2), whilst any position of the plug (14) proves directly visible for the user through the front grill.

The form of the front grill, allows perfect viewing of the regulator plug element and with it a view is quickly obtained of the degree of evaporation and diffusion of the device.

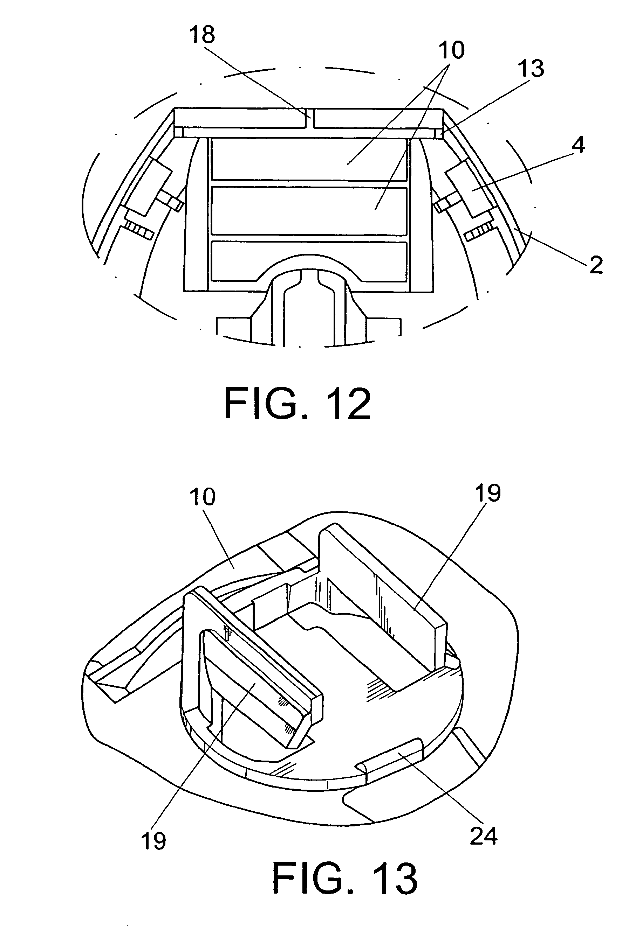

As an addition to the structure described, the rear half-casing (2) incorporates, under its grill (10), a kind of fork (19), based on two arms elbowed backward and downward, as may be especially observed in the detail of figure 13, between which arms can be coupled the side webs (20) of a metallic band constituting the clip for fastening the diffuser, which arms are each finished in jackets (21) of plastic material, with the particularity that from the core (22), from which the mentioned elastic arms (20) of the clip emerge in opposition, two other arms (23) emerge, offset 90° with respect to the previous ones, likewise convergent but considerably shorter, so that the clip as an assembly can be coupled to the fork (19), in a matched engagement and by means of a slight elastic deformation, in two different positions and rotated by 90°, so that the clip (21) can be positioned in the horizontal direction and the vertical direction, according to the specific necessities of each case, collaborating in any case in the stabilization of the core (22) in its selected working position, a small rib (24) present on the wall of the rear half-casing (2) and properly facing the mouth of the fork (19), which rib should be surpassed by the core (22), also by means of a slight elastic deformation, in its advance toward its assembled position.

## Claims

1. Diffuser of air-freshener products for vehicles, comprising a casing (1,2) and a container (7) of an air-freshening product coupled to said casing, said casing having grills (9,10) facing each other for the passage of air through the casing,
wherein said container (7) is provided with a wick (8) for the evaporation of said product, wherein a part of said wick is housed inside the casing between said grills (9,10),
wherein the casing comprises two cylindrical and coaxial necks, a lower neck (5) adapted to receive tightly a neck (6) of said container (7), and an upper neck (12) in which a plug (14) is rotatably mounted, said plug (14) having a handle (16) projecting outwardly from the casing for its manual operation,
**characterised in that** the plug (14) consists of a pair of cylindrical skirts (17) joined to said handle (16) and arranged in a diametrical opposition inside the casing (1,2), so that
said plug (14) is rotatable from a first position in which the grills (9,10) are open, to a second position in which the grills (9,10) are fully closed by said cylindrical skirts (17).

2. Diffuser of air-freshening products for vehicles according to claim 1 wherein the casing is provided with fastening means for fastening the diffuser to a diffuser grill of the air-conditioning equipment of a vehicle.

3. - Diffuser of air-freshening products for vehicles, according to claim 1 or claim 2, **characterised in that** its casing is structured by means of two half-casings, a front one (1) and another rear one (2), capable of matched engagement by means of legs (3) present on one of them, which are coupled in complementary vents (4) of the other one, the half of each one of the upper (12) and lower (5) necks being defined in each one of said half-casings, and the upper neck (12) incorporating an internal peripheral rib (13) which likewise fits in a peripheral groove (15) of the plug (14), allowing the rotation of the latter but immobilizing it axially, with the particularity that said groove (15) has a widened sector (13'), in which runs a butt (18) pertaining to the rear casing (2), acting as rotation constraint for said plug (14) and as means determining the end positions of opening and closing for the same.

4. - Diffuser of air-freshening products for vehicles, according to claim 3, **characterised in that** one of the two skirts (17) of the plug, is visible through the front grill (9) of the front half-casing (1), showing its position of full or partial opening, as well as its closing position.

5. Diffuser of air-freshening products for vehicles, according to previous claims, **characterised in that** one of the two skirts (17) of the plug (14) is cut, so that the length of this cut skirt (17) is approximately three times less than the length of the other skirt (17).

6. Diffuser of air-freshening products for vehicles, according to claim 3, **characterised in that** the rear half-casing (2) incorporates, below its grill (10), a protuberance (19) of forked configuration, for fastening a clip implemented in a band in which participate two arms (20), in opposition, with jackets (21) of plastic nature, which arms (20) emerge from a flat common core (22), from which in turn emerge other two arms (23), offset by 90° with respect to the previous ones and considerably shorter, which like these last ones are markedly convergent for their matched engagement with the fork (19) of the rear half-casing, so that the clip can be coupled by press-fitting on the fork (19), in two positions rotated through 90°, corresponding to a horizontal or vertical positioning of the actual clip, indistinctly, collaborating in the fastening of said clip a stop (24) established in the rear wall of the rear half-casing (2), facing the mouth of the fork (19).

## Patentansprüche

1. Diffuser für Lufterfrischungsprodukte für Fahrzeuge, umfassend ein Gehäuse (1, 2) und einen Behälter (7) eines Lufterfrischungsprodukts, der mit dem Gehäuse verbunden ist, wobei das Gehäuse (9, 10) Gitter zum Durchlassen von Luft durch das Gehäuse aufweist, die einander zugewandt sind,
wobei der Behälter (7) mit einem Docht (8) zur Verdunstung von dem Produkt versehen ist, wobei ein Abschnitt des Dochts in dem Gehäuse zwischen den Gittern (9, 10) untergebracht ist,
wobei das Gehäuse zwei zylindrische und koaxiale Ansätze aufweist, einen unteren Ansatz (5), der ausgebildet ist, um einen Ansatz (6) des Behälters (7) eng aufzunehmen und einen oberen Ansatz (12) in dem ein Stecker (14) drehbar angeordnet ist, wobei der Stecker (14) einen Griff (16) aufweist, der sich zu seiner manuellen Betätigung aus dem Gehäuse erstreckt,
**dadurch gekennzeichnet, dass**
der Stecker (14) aus einem Paar zylindrischer Einfassungen (17) besteht, die mit dem Griff (16) verbunden sind und diametral gegenüberliegend in dem Gehäuse (1, 2) angeordnet sind, so dass
der Stecker (14) von einer ersten Position, in der die Gitter (9, 10) geöffnet sind, in eine zweite Position, in der die Gitter (9, 10) vollständig von den Einfassungen verschlossen sind, drehbar ist.

2. Diffuser für Lufterfrischungsprodukte für Fahrzeuge nach Anspruch 1, bei dem das Gehäuse mit Befestigungsmitteln zum Befestigen des Diffusors an einem Diffusorgitter der Klimaanlageneinrichtung eines Fahrzeuges versehen ist.

3. Diffuser für Lufterfrischungsprodukte für Fahrzeuge nach Anspruch 1 oder 2, **dadurch gekennzeichnet, dass** das Gehäuse mittels zweier Halbgehäuse gegliedert ist, ein vorderes (1) und ein hinteres (2), die mittels an einem von ihnen vorliegenden Füßen (3) zur zusammenpassenden Verbindung in der Lage sind, wobei die Füße (3) mit komplementären Öffnungen (4) des anderen verbunden sind, wobei die Hälfte von jeweils dem oberen (12) und dem unteren (5) in jedem der Halbgehäuse festgelegt ist und der obere Ansatz (12) eine interne Umfangsrippe (13) aufnimmt, die gleichermaßen in eine Umfangsnut (15) des Steckers (14) passt, wodurch die Drehung des letzteren ermöglicht wird, er aber axial ruhig gestellt wird, mit der Besonderheit, dass die Nut (15) einen erweiterten Abschnitt (13') aufweist, durch den ein Stumpf (18) verläuft, der zu dem hinteren Gehäuse (2) gehört, als Drehbegrenzer für den Stecker (14) dient und als Mittel zum Ermitteln der Endpositionen des Öffnens und des Schließens desselbigen.

4. Diffuser für Lufterfrischungsprodukte für Fahrzeuge nach Anspruch 3, **dadurch gekennzeichnet, dass** eine der beiden Einfassungen (17) des Steckers durch das vordere Gitter (9) des vorderen Halbgehäuses (1) sichtbar ist, wodurch sowohl deren volle oder teilweise Öffnungsposition als auch deren Schließposition gezeigt wird.

5. Diffuser für Lufterfrischungsprodukte für Fahrzeuge nach einem der vorhergehenden Ansprüche, **dadurch gekennzeichnet, dass** eine der beiden Einfassungen (17) des Steckers (14) abgeschnitten ist, so dass die Länge der abgeschnittenen Einfassung (17) ungefähr dreimal weniger beträgt als die Länge der anderen Einfassung (17).

6. Diffusor für Lufterfrischungsprodukte für Fahrzeuge nach Anspruch 3, **dadurch gekennzeichnet, dass** das hintere Halbgehäuse (2) unterhalb seines Gitters (10) einen gabelförmig aufgebauten Vorsprung (19) zum Befestigen eines Clips umfasst, wobei der Clip in einem Band umgesetzt ist in dem zwei gegenüberliegende Arme (20) mit Ummantelungen (21) aus plastikhaltigem Ursprung mitwirken, wobei die Arme (20) sich von einem flachen gemeinsamen Kern (22) aus erstrecken, von dem sich wiederum zwei weitere in Bezug auf die vorherigen Arme um 90° versetzte, deutlich kürzere Arme (23) erstrecken, wobei die letzteren merklich zu ihrer passenden Verbindung mit der Gabel (19) des hinteren Halbgehäuses zusammenlaufen, so dass der Clip durch Presspassung auf die Gabel (19) in zwei um 90° verdrehten Positionen, die einer horizontalen oder vertikalen Positionierung des tatsächlichen Clips entsprechen gekoppelt werden kann, wodurch unklar bei der Befestigung des Clips ein Anschlag (24) in der hinteren Wand des hinteren Halbgehäuses (2), die auf den Mund der Gabel (19) zeigt, geschaffen wird.

## Revendications

1. Diffuseur de produits désodorisants pour véhicules, comprenant un boîtier (1, 2) et un récipient (7) d'un produit désodorisant couplé audit boîtier, ledit boîtier ayant des grilles (9, 10) se faisant face pour le passage de l'air à travers le boîtier,
dans lequel ledit récipient (7) est doté d'une mèche (8) pour l'évaporation dudit produit, dans lequel une partie de ladite mèche est logée à l'intérieur du boîtier entre lesdites grilles (9, 10),
dans lequel ledit boîtier comprend deux cols cylindriques et coaxiaux, un col inférieur (5) adapté pour recevoir de manière serrée un col (6) dudit récipient (7), et un col supérieur (12) dans lequel un bouchon (14) est monté de manière rotative, ledit bouchon (14) ayant une poignée (16) faisant saillie vers l'extérieur à partir du boîtier pour son actionnement manuel,
**caractérisé en ce que** le bouchon (14) se compose d'une paire de collerettes cylindriques (17) assemblées à ladite poignée (16) et agencées selon une opposition diamétrale à l'intérieur du boîtier (1, 2), de sorte que :
ledit bouchon (14) puisse tourner d'une première position dans laquelle les grilles (9, 10) sont ouvertes jusqu'à une seconde position dans laquelle les grilles (9, 10) sont complètement fermées par lesdites collerettes cylindriques (17).

2. Diffuseur de produits désodorisants pour véhicules selon la revendication 1, dans lequel le boîtier est doté de moyens de fixation pour fixer le diffuseur sur une grille de diffuseur de l'équipement de conditionnement d'air d'un véhicule.

3. Diffuseur de produits désodorisants pour véhicules, selon la revendication 1 ou la revendication 2, **caractérisé en ce que** son boîtier est structuré au moyen de deux demi-boîtiers, un boîtier avant (1) et un autre boîtier arrière (2), pouvant réaliser une mise en prise correspondante au moyen de pattes (3) présentes sur l'un d'entre eux, qui sont couplées dans des conduits d'aération complémentaires (4) de l'autre, la moitié de chacun des cols supérieur (12) et inférieur (5) étant définie dans chacun desdits demi-boîtiers, et le col supérieur (12) comprenant une nervure périphérique interne (13) qui se monte de même dans une rainure périphérique (15) du bouchon (14) permettant la rotation de ce dernier mais l'immobilisant de manière axiale, avec la particularité que ladite rainure (15) a un secteur élargi (13'), dans lequel s'étend une butée (18) appartenant au boîtier arrière (2), servant de contrainte de rotation pour ledit bouchon (14) et de moyen pour déterminer les positions d'extrémité d'ouverture et de fermeture de ce dernier.

4. Diffuseur de produits désodorisants pour véhicules, selon la revendication 3, **caractérisé en ce que** l'une des deux collerettes (17) du bouchon, est visible à travers la grille avant (9) du demi-boîtier avant (1), dévoilant sa position d'ouverture complète ou partielle, ainsi que sa position de fermeture.

5. Diffuseur de produits désodorisants pour véhicules, selon l'une quelconque des revendications précédentes, **caractérisé en ce que** l'une des deux collerettes (17) du bouchon (14) est coupée, de sorte que la longueur de cette collerettes coupée (17) représente approximativement trois fois moins que la longueur de l'autre collerettes (17).

6. Diffuseur de produits désodorisants pour véhicules, selon la revendication 3, **caractérisé en ce que** le demi-boîtier arrière (2) comprend, au-dessous de sa grille (10), une protubérance (19) de configuration fourchue, pour fixer une attache mise en oeuvre dans une bande dans laquelle participent deux bras (20), en opposition avec des chemises (21) en plastique, lesquels bras (20) sortent d'un noyau commun plat (22) à partir duquel sortent à leur tour deux autres bras (23), décalés de 90° par rapport aux précédents et considérablement plus courts, qui comme ces derniers, sont considérablement convergents pour leur mise en prise correspondante avec la fourche (19) du demi-boîtier arrière, de sorte que l'attache puisse être couplée par un ajustement à force sur la fourche (19), dans deux positions décalées de 90°, correspondant à un positionnement horizontal ou vertical de la véritable attache, collaborant indistinctement dans la fixation de ladite attache, une butée (24) établie dans la paroi arrière du demi-boîtier arrière (2), faisant face à l'embouchure de la fourche (19).
